(19) Europäisches Patentamt
European Patent Office
Office européen des brevets

(11) EP 2 098 266 B2

(12) **NOUVEAU FASCICULE DE BREVET EUROPEEN**
Après la procédure d'opposition

(45) Date de publication et mention de la décision concernant l'opposition:
**26.08.2015 Bulletin 2015/35**

(45) Mention de la délivrance du brevet:
**21.12.2011 Bulletin 2011/51**

(21) Numéro de dépôt: **08168552.1**

(22) Date de dépôt: **07.11.2008**

(51) Int Cl.:
***A61Q 5/10*** (2006.01) ***A61K 8/73*** (2006.01)

(54) **Composition comprenant un dérivé de cellulose modifié, un polymère non ionique et des colorants d'oxydation, procédé de teinture d'oxydation et d'utilisation**

Zusammensetzung, die ein modifiziertes Zellulosederivat, ein nicht-ionisches Polymer und Oxydationsfarbstoffe umfasst, Oxydationsfärbe- und Anwendungsverfahren

Composition comprising a modified cellulose derivative, a non-ionic polymer and oxidising dyes, dyeing method for oxidation and use.

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **09.11.2007 FR 0758912**

(43) Date de publication de la demande:
**09.09.2009 Bulletin 2009/37**

(73) Titulaire: **L'Oréal**
**75008 Paris (FR)**

(72) Inventeur: **Audousset, Marie-Pascale**
**92600 ASNIERES (FR)**

(74) Mandataire: **Casalonga, Axel**
**Casalonga & Partners**
**Bayerstrasse 73**
**80335 München (DE)**

(56) Documents cités:
**EP-A- 1 426 038** **EP-A- 1 426 040**
**EP-A- 1 473 025** **EP-A- 1 566 164**
**EP-A- 1 707 190** **EP-A1- 1 036 558**
**EP-A1- 1 992 329** **EP-A1- 2 915 883**
**EP-A2- 0 412 705** **WO-A-98/03150**
**WO-A2-99/11223** **WO-A2-02/051372**

EP 2 098 266 B2

## Description

La présente demande a pour objet une composition de teinture d'oxydation des fibres kératiniques, et en particulier des fibres kératiniques humaines telles que les cheveux, comprenant un ou plusieurs dérivé(s) non ionique(s) de cellulose modifié(s) par un ou plusieurs groupement(s) hydrophobe(s) particulier(s), un ou plusieurs polymère(s) non hydrophobe(s) non ionique(s) de type hydroxyalkylcellulose, et un ou plusieurs colorant(s) d'oxydation.

L'invention a aussi pour objet l'utilisation de cette composition pour la teinture des fibres kératiniques ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences.

Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (i.e. abîmées) de sa pointe à sa racine.

Par ailleurs, les compositions obtenues doivent, en outre, présenter de bonnes propriétés rhéologiques, tout en conservant de bonnes propriétés de coloration. En particulier, ces compositions ne doivent pas couler sur le visage ou en dehors des zones que l'on se propose de teindre, lors de leur application, notamment après mélange avec un agent oxydant.

Il est déjà connu de la demande WO 98/03150 d'améliorer la puissance de la coloration en associant une base d'oxydation para-phénylènediamine et au moins un polymère amphiphile non ionique du type hydroxycellulose modifiée par un groupement hydrophobe.

Le document EP-A-1 426 040 divulgue l'association d'hydroxypropyl methyl cellulose, de polyuréthane cationique associatif et de colorants d'oxydation particuliers.

Cependant, ces compositions ne satisfont pas pleinement les exigences précitées et peuvent être améliorées, notamment en termes de propriétés tinctoriales, en particulier au niveau de la sélectivité et des ténacités. Le but de la présente invention est l'obtention de compositions de coloration capillaire stables, notamment sous forme de crèmes, faciles à préparer et à appliquer, ayant de bonnes qualités rhéologiques et conduisant à des colorations intenses, peu sélectives et résistantes aux diverses agressions que peuvent subir les fibres kératiniques.

Ce but est atteint par la présente invention qui a pour objet une composition tinctoriale pour fibres kératiniques, et en particulier pour fibres kératiniques humaines telles que les cheveux, comprenant, dans un milieu approprié pour la teinture :

(A) un ou plusieurs dérivé(s) non ionique(s) de cellulose comprenant un ou plusieurs substituant(s) hydrophobe(s) comprenant de 8 à 30 atomes de carbone ;

(B) un ou plusieurs polymère(s) non hydrophobe(s) non ionique(s) de type hydroxyalkylcellulose ; et

(C) un ou plusieurs colorant(s) d'oxydation choisi(s) parmi les bases d'oxydation suivantes : les para-phénylènediamines, les bis-phénylalkylènediamines, les para-aminophénols, les bis-para-aminophénols, les ortho-aminophénols, et leurs sels d'addition.

Les compositions tinctoriales selon l'invention présentent notamment les propriétés suivantes :

- elles permettent d'obtenir des compositions de viscosité correspondant à une crème qui sont stables dans le temps,
- elles se distinguent par une facilité de mélange avec la composition oxydante,
- elles se distinguent par les qualités rhéologiques des crèmes obtenues (bonne viscosité de crème en mélange),
- elles sont faciles à appliquer après mélange avec la composition oxydante au moment de la mise en oeuvre de la

coloration (qualités d'usage sur tête).

En outre, les compositions selon l'invention permettent l'obtention de compositions capables de conduire à des colorations aux nuances variées, chromatiques, puissantes, esthétiques, peu sélectives, uniformes sur l'ensemble des fibres kératiniques et en particulier des fibres kératiniques humaines telles que les cheveux, et résistant bien aux diverses agressions que peuvent subir les fibres.

Un autre objet de la présente invention consiste en un procédé de teinture des fibres kératiniques dans lequel la composition cosmétique selon l'invention est mise en oeuvre.

Un troisième objet de l'invention concerne l'utilisation de cette composition cosmétique pour la teinture des fibres kératiniques, et en particulier des fibres kératiniques humaines, telles que les cheveux.

D'autres caractéristiques, aspects, objets et avantages de la présente invention apparaîtront encore plus clairement à la lecture de la description et des exemples qui suivent.

A moins d'une indication différente, les bornes des gammes de valeurs qui sont données dans le cadre de la présente invention sont incluses dans ces gammes.

Par « dérivé(s) de cellulose », on entend un (ou des) composé(s) comportant au moins un motif cellobiose de structure suivante :

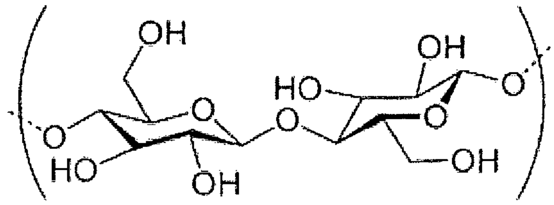

dans laquelle, un ou plusieurs groupe(s) hydroxyle peut (ou peuvent) être substitué(s).

Le ou les dérivés non ioniques de cellulose à substituant(s) hydrophobe(s) (A) conformes à la présente invention, sont des polymères amphiphiles présentant un caractère associatif. En effet, ils comprennent des motifs hydrophiles et des motifs hydrophobes et sont capables d'interagir et de s'associer entre eux ou à d'autres molécules, de manière réversible, en particulier, grâce à la présence de leurs chaînes hydrophobes.

De préférence, le dérivé de cellulose de l'invention est un éther de cellulose comprenant un ou plusieurs substituant(s) hydrophobe(s) comprenant de 8 à 30 atomes de carbone.

Le ou les dérivés non ioniques de cellulose à substituant(s) hydrophobe(s) conformes à la présente invention, sont préparés généralement à partir d'éthers non-ioniques de cellulose hydrosolubles, dont on substitue tout ou partie des fonctions hydroxyle réactives par une ou plusieurs chaîne(s) hydrophobe(s) comprenant de 8 à 30 atomes de carbone, de préférence de 10 à 22 atomes de carbone, et mieux encore 16 atomes de carbone. Les étapes de réactions en jeu dans la préparation des dérivés de cellulose de l'invention sont connues de l'homme du métier.

Les éthers non-ioniques de cellulose choisis pour préparer les dérivés non-ioniques de cellulose à substituant(s) hydrophobe(s) selon l'invention, ont de préférence un degré de substitution non-ionique, par exemple en groupement(s) méthyle, hydroxyéthyle ou hydroxypropyle, suffisant pour être hydrosolubles, c'est-à-dire former une solution sensiblement limpide lorsqu'ils sont dissous dans l'eau à 25 °C à la concentration de 1 % en poids.

Les éthers non ioniques de cellulose choisis pour préparer les dérivés non ioniques de cellulose à substituant(s) hydrophobe(s) selon l'invention, possèdent préférentiellement une masse molaire moyenne en nombre relativement faible, inférieure à 800 000 g/mole, de préférence allant de 50 000 et 700 000 g/mole et de manière plus préférée allant de 200 000 à 600 000 g/mole.

De préférence, le dérivé de cellulose de l'invention est une hydroxyéthylcellulose comprenant un ou plusieurs substituant(s) hydrophobe(s) comprenant de 8 à 30 atomes de carbone.

Les dérivés non-ioniques de cellulose utilisés selon l'invention sont substitués , par une ou plusieurs chaîne(s) hydrocarbonée(s) en $C_8$-$C_{30}$ linéaires, ramifiées ou cycliques, saturées ou insaturées, aliphatiques ou aromatiques, pouvant être rattachées au substrat éther de cellulose par une liaison éther, ester, ou uréthane, de préférence éther.

Selon un mode de réalisation, le ou les substituants hydrophobes utilisés comme substituants des dérivés non-ioniques de cellulose selon la présente invention sont des groupes alkyle, arylalkyle ou alkylaryle en $C_8$-$C_{30}$, de préférence en $C_{10}$-$C_{22}$.

De préférence, le ou les substituants hydrophobes selon la présente invention sont des chaînes alkyles saturées.

Selon un mode de réalisation préféré, le ou les substituants hydrophobes selon la présente invention sont des groupements cétyle.

Les dérivés non-ioniques de cellulose à substituant(s) hydrophobe(s) selon l'invention, présentent une viscosité de préférence comprise entre 100 et 100 000 mPa.s, et de préférence entre 200 et 20 000 mPa.s, mesurée à 25 °C

dans une solution à 1 % en poids de polymère dans l'eau, cette viscosité étant déterminée de manière conventionnelle à l'aide d'un viscosimètre de type Brookfield LVT à 6 tours par minute avec le mobile n° 3.

Le degré de substitution hydrophobe des dérivés non-ioniques de cellulose hydrophiles utilisés selon l'invention, va préférentiellement de 0,1 à 10 % en poids, plus préférentiellement de 0,1 à 1 % en poids et de manière particulièrement préférée de 0,4 à 0,8 % en poids, du poids total du polymère.

Parmi les dérivés non-ioniques de cellulose à substituant(s) hydrophobe(s) utilisables dans les compositions de l'invention, on peut citer, de préférence, les cétyl hydroxyéthylcelluloses commercialisées sous les dénominations Natrosol Plus Grade 330 CS et Polysurf 67 CS (INCI : Cetyl Hydroxyethylcellulose) par la société Aqualon/Hercules.

La concentration en dérivé(s) non-ionique(s) de cellulose à substituant(s) hydrophobe(s) (A) des compositions selon l'invention va de préférence de 0,01 à 10 % en poids, en particulier de 0,05 à 3 % en poids, et de manière plus préférée de 0,1 à 1 % en poids, par rapport au poids total de la composition.

Par « polymère non hydrophobe » on entend au sens de la présente invention un polymère ne comprenant pas dans sa structure de chaîne grasse comportant au moins 8 atomes de carbone.

Le ou les polymère(s) non hydrophobe(s) non ionique(s) de type hydroxyalkylcellulose (B) utilisable(s) selon l'invention sont préparés à partir de cellulose dont on éthérifie, de manière bien connue de l'homme du métier, tout ou partie des fonctions hydroxyle par un ou plusieurs groupement(s) alkyle(s) ou hydroxyalkyle(s), identiques ou différents, comprenant de préférence de 1 à 4 atomes de carbone.

Le ou les polymère(s) non hydrophobe(s) non ionique(s) de type hydroxyalkylcellulose (B) utilisable(s) selon l'invention comprennent ainsi un ou plusieurs groupement(s) alkyle(s) ou hydroxyalkyle(s), identiques ou différents, linéaire(s) ou ramifié(s), saturé(s) ou insaturé(s), ayant de préférence de 1 à 4 atomes de carbone.

A titre de groupement(s) alkyle(s) ou hydroxyalkyle(s) ayant de 1 à 4 atomes de carbones utilisable(s), on peut citer les groupements méthyle, éthyle, propyle, isopropyle, hydroxyméthyle, hydroxyéthyle, hydroxypropyle et hydroxyisopropyle.

Le polymère non hydrophobe non ionique de type hydroxyalkylcellulose (B) selon l'invention est de préférence choisi parmi :

- les hydroxypropylméthylcelluloses, telles que celles commercialisées sous la dénomination METHOCEL F4M par la société Dow Chemical,
- les hydroxyéthylcelluloses, telles que celles commercialisées sous la dénomination NATROSOL 250 HHR par la société Aqualon.

Le polymère non hydrophobe non ionique de type hydroxyalkylcellulose (B) particulièrement préféré selon l'invention est l'hydroxypropylméthylcellulose.

La concentration en polymère(s) non hydrophobe(s) non ionique(s) de type hydroxyalkylcellulose (B) des compositions selon l'invention va de préférence de 0,01 à 5 %, en particulier de 0,05 à 2 % en poids, et de manière plus préférée de 0,1 à 1 % en poids, par rapport au poids total de la composition.

Les bases d'oxydation utilisables sont choisies parmi les para-phénylènediamines, les bis-phénylalkylènediamines, les para-aminophénols, les bis-para-aminophénols, les ortho-aminophénols et leurs sels d'addition.

Parmi les para-phénylènediamines, on peut citer à titre d'exemple, la para-phénylènediamine, la para-toluylènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino-2-méthyl aniline, la 4-N,N-bis-(β-hydroxyéthyl)amino-2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylène-diamine, la 4-aminophénylpyrrolidine, la 2-thiényl para-phénylènediamine, le 2-β hydroxyéthylamino-5-aminotoluène, la 3-hydroxy-1-(4'-aminophényl)pyrrolidine et leurs sels d'addition avec un acide.

Parmi les para-phénylènediamines citées ci-dessus, la para-phénylènediamine, la para-toluylènediamine, la 2-isopropyl para-phénylènediamine, la 2-β-hydroxyéthyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino-3-méthyl phénol, le 4-amino-3-fluoro phénol, le 4-amino-3-hydroxyméthyl phénol, le 4-amino-2-méthyl phénol, le 4-amino-2-hydroxyméthyl phénol, le 4-amino-2-méthoxyméthyl phénol, le 4-amino-2-aminométhyl phénol, le 4-amino-2-(β-hydroxyéthyl aminométhyl)phénol, le 4-amino 2-fluoro phénol, et leurs sels d'addition avec un acide.

Parmi les ortho-aminophénols, on peut citer à titre d'exemple, le 2-aminophénol, le 2-amino-5-méthylphénol, le 2-amino-6-méthylphénol, le 5-acétamido-2-aminophénol, et leurs sels d'addition avec un acide.

La concentration en base(s) d'oxydation va en général de 0,001 à 20 % en poids, de préférence de 0,005 à 10 % en poids, et encore plus préférentiellement de 0,01 à 5 % en poids, par rapport au poids total de la composition.

Le (ou les) coupleur(s) d'oxydation pouvant être présent(s) dans les compositions de l'invention, peut (ou peuvent) être choisi(s) parmi les coupleurs benzéniques, les coupleurs hétérocycliques, les coupleurs naphtaléniques, et leurs sels d'addition.

A titre de coupleurs benzéniques utilisables dans les compositions selon l'invention, on peut citer les méta-aminophénols, les méta-phénylènediamines, les méta-diphénols, ainsi que leurs sels d'addition.

Parmi les coupleurs préférés, on peut citer le 2-méthyl-5-aminophénol, le 5-N-(β-hydroxyéthyl)amino-2-méthyl phénol, le 6-chloro-2-méthyl-5-aminophénol, le 3-aminophénol, le 1,3-dihydroxy benzène, le 1,3-dihydroxy-2-méthyl benzène, le 4-chloro-1,3-dihydroxy benzène, le 2,4-diamino-1-(β-hydroxyéthyloxy)benzène, le 2-amino-4-(β-hydroxyéthylamino)-1-méthoxybenzène, le 1,3-diamino benzène, le 1,3-bis-(2,4-diaminophénoxy)propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

La concentration en coupleur(s) d'oxydation va en général de 0,001 à 20 % en poids, de préférence de 0,005 à 10 % en poids, et encore plus préférentiellement de 0,01 à 5 % en poids, par rapport au poids total de la composition.

Selon un mode de réalisation particulier, les compositions tinctoriales de l'invention comprennent, dans un milieu approprié pour la teinture :

(A) un ou plusieurs dérivé(s) non ionique(s) de cellulose comprenant un ou plusieurs substituant(s) hydrophobe(s) comprenant de 8 à 30 atomes de carbone tel(s) que défini(s) précédemment ;
(B) un ou plusieurs polymère(s) non hydrophobe(s) non ionique(s) de type hydroxyalkylcellulose ; et
(C) une ou plusieurs base(s) d'oxydation telles que définies ci-avant et un ou plusieurs coupleur(s) d'oxydation.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut, en outre, contenir un ou plusieurs colorant(s) direct(s) pouvant notamment être choisi(s) parmi les colorants nitrés benzéniques, les colorants directs azoïques, les colorants directs méthiniques, les colorants anthraquinoniques, les colorants xanthéniques, les colorants triarylméthaniques et leurs sels d'addition. Ces colorants directs peuvent être de nature non ionique, anionique ou cationique.

Le milieu utilisé dans les compositions selon la présente invention est un milieu aqueux ou un milieu contenant de l'eau et au moins un solvant organique.

Le (ou les) solvant(s) organique(s) utilisé(s) dans les compositions selon la présente invention peut (ou peuvent) être choisi(s) parmi les alcools monohydroxylés et les polyols.

A titre d'alcools monohydroxylés utilisables, on peut citer les alcools inférieurs en $C_1$-$C_4$ comme l'éthanol, l'isopropanol, le tertiobutanol, le n-butanol, et leurs mélanges. De préférence, l'alcool utilisé est l'éthanol.

À titre de polyols utilisables, on peut citer le propylèneglycol, les polyéthylèneglycols, la glycérine. A titre de solvants organiques, on peut aussi citer les éthers de polyols comme le 2-butoxyéthanol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

La concentration en solvant(s) organique(s) dans les compositions selon la présente invention est comprise de préférence entre 0 et 30 %, et de manière plus préférée entre 0 et 20 % en poids par rapport au poids total de la composition.

Les compositions selon la présente demande peuvent également contenir, un ou plusieurs agent(s) épaississant(s) encore appelé "agent(s) d'ajustement de la rhéologie" différents des dérivés non-ioniques de cellulose à substituant(s) hydrophobe(s) de l'invention.

L'agent (ou les agents) d'ajustement de la rhéologie peut (ou peuvent) être choisi(s) parmi les agents épaississants minéraux ou organiques, et en particulier les épaississants associatifs polymériques, les alcools gras (alcool oléïque), les dérivés cellulosiques autres que les dérivés de cellulose non-ioniques à substituant(s) hydrophobe(s) (A) et les polymères non hydrophobes non ioniques de type hydroxyalkylcellulose (B) selon l'invention (carboxyméthylcellulose) et les gommes d'origine microbienne (gomme de xanthane, gomme de scléroglucane).

L'agent (ou les agents) d'ajustement de la rhéologie préféré(s) est (ou sont) choisi(s) parmi les alcools gras notamment en $C_{20}$-$C_{22}$ et les dérivés de celluloses, autre que les dérivés de cellulose non-ioniques à substituant(s) hydrophobe(s) (A) et les polymères non hydrophobes non ioniques de type hydroxyalkylcellulose (B) selon l'invention.

La concentration en agent(s) épaississant(s) est comprise de préférence entre 0,01 et 20 % en poids, et de manière plus préférée entre 1 et 10 % en poids, par rapport au poids total de la composition

La composition tinctoriale conforme à l'invention peut également contenir un ou plusieurs adjuvant(s) utilisé(s) classiquement dans les compositions pour la teinture des cheveux.

Par « adjuvant(s) », on entend un (ou des) additif(s), différent(s) des composés précités, tels que les agents tensioactifs anioniques, cationiques, non-ioniques, amphotères, zwittérioniques ou leurs mélangés ; les polymères non-ioniques, amphotères, zwittérioniques, anioniques, cationiques autres que les dérivés de cellulose non-ioniques à substituant(s) hydrophobe(s) (A) et les polymères non hydrophobe non ionique de type hydroxyalkylcellulose (B) selon l'invention, ou leurs mélanges ; les agents de pénétration ; les agents séquestrants ; les parfums ; les tampons ; les agents dispersants ; les agents conditionneurs tels que par exemple de silicones volatiles ou non volatiles, modifiées ou non modifiées ; des agents filmogènes ; les céramides ; les agents conservateurs ; les agents opacifiants ; les vitamines ; les acides aminés ; les oligopeptides ; les peptides ; les protéines hydrolysées ou non, modifiées ou non ; les enzymes ; les acides et alcools gras ramifiés ou non ; les cires animales, végétales ou minérales ; les acides organiques hydroxylés ; les filtres UV ; les agents anti-oxydants et les agents anti-radicaux libres ; les agents antipelliculaires ; les agents régulateurs de séborrhée ; les agents apaisants ; les huiles minérales, végétales ou animales ; les polyisobutènes et poly($\alpha$-oléfines) ; les pigments ; les acides, bases, plastifiants, charges minérales, nacres, paillettes ; les agents anti-statiques et les agents réducteurs.

Le (ou les) adjuvant(s) ci-dessus est (ou sont), en général, présent(s) en quantité comprise, pour chacun d'eux, de préférence entre 0,01 et 40 % en poids, et de manière plus préférée entre 0,1 et 25 % en poids par rapport au poids de la composition.

Bien entendu, l'homme de l'art veillera à choisir ce (ou ces) éventuel(s) composé(s) complémentaire(s) de manière telle que les propriétés avantageuses attachées intrinsèquement à la composition de teinture d'oxydation conforme à l'invention ne soient pas, ou substantiellement pas, altérées par la (ou les) adjonction(s) envisagée(s).

Le pH de la composition tinctoriale conforme à l'invention va généralement de 3 à 12 environ, et de préférence de 5 à 11 environ. Il peut être ajusté à la valeur désirée au moyen d'agent(s) acidifiant(s) ou alcalinisant(s), habituellement utilisés en teinture des fibres kératiniques ou bien encore à l'aide de système(s) tampon(s) classique(s).

Parmi les agents acidifiants, on peut citer, à titre d'exemple, les acides minéraux ou organiques comme l'acide chlorhydrique, l'acide orthophosphorique, l'acide sulfurique, les acides sulfoniques et les acides carboxyliques comme l'acide acétique, l'acide tartrique, l'acide citrique et l'acide lactique.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (I) suivante :

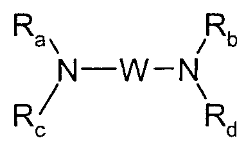

(I)

dans laquelle :

- W est un reste propylène éventuellement substitué par un groupe hydroxyle ou un groupe alkyle en $C_1$-$C_4$ ;
- $R_a$, $R_b$, $R_c$ et $R_d$, identiques ou différents, représentent un atome d'hydrogène, un groupe alkyle en $C_1$-$C_4$ ou hydroxyalkyle en $C_1$-$C_4$.

La composition tinctoriale selon l'invention peut se présenter sous des formes diverses, telles que sous forme de crèmes, de gels, ou sous toute autre forme appropriée pour réaliser une teinture des fibres kératiniques, et notamment

des cheveux humains.

Le procédé de teinture des fibres kératiniques de la présente invention est un procédé dans lequel on applique sur les fibres la composition selon la présente invention telle que définie précédemment, de préférence en présence d'au moins un agent oxydant, pendant un temps suffisant pour développer la couleur désirée. La couleur peut être révélée à pH acide, neutre ou alcalin et l'agent (ou les agents) oxydant(s) peut (ou peuvent) être ajouté(s) à la composition de l'invention juste au moment de l'emploi ou il(s) peut (ou peuvent) être mis en oeuvre à partir d'une composition oxydante le(s) contenant, appliquée simultanément ou séquentiellement à la composition de l'invention.

Selon un mode de réalisation particulier, la composition selon la présente invention est une composition prête à l'emploi, mélangée, de préférence au moment de l'emploi, à une composition contenant, dans un milieu approprié pour la teinture, au moins un agent oxydant, cet agent (ou ces agents) oxydant(s) étant présent(s) en une quantité suffisante pour développer une coloration. Le mélange obtenu est ensuite appliqué sur les fibres kératiniques. Après un temps de pause de 3 à 50 minutes environ, de préférence, 5 à 30 minutes environ, les fibres kératiniques sont rincées, lavées au shampooing, rincées à nouveau, puis séchées.

Les agents oxydants classiquement utilisés pour la teinture d'oxydation des fibres kératiniques sont, par exemple, le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, ces oxydoréductases étant éventuellement associées à leurs cofacteurs habituels tels que l'acide urique pour les uricases. L'agent oxydant préféré est le peroxyde d'hydrogène.

La composition oxydante peut également renfermer divers adjuvants utilisés classiquement dans les compositions pour la teinture des cheveux, tels que définis précédemment.

Le pH de la composition oxydante renfermant l'agent oxydant est tel qu'après mélange avec la composition tinctoriale, le pH de la composition résultante appliquée sur les fibres kératiniques varie, de préférence, de 3 à 12 environ, et préférentiellement, de 5 à 10. Il peut être ajusté à la valeur désirée au moyen d'agent(s) acidifiant(s) ou alcalinisant(s) habituellement utilisé(s) en teinture des fibres kératiniques, tel(s) que défini(s) précédemment.

La composition prête à l'emploi qui est finalement appliquée sur les fibres kératiniques, peut se présenter sous des formes diverses, telles que sous forme de crèmes, de gels ou sous toute autre forme appropriée pour réaliser une teinture des fibres kératiniques, et notamment des fibres kératiniques humaines tels que les cheveux.

L'invention a aussi pour objet un dispositif à plusieurs compartiments ou "kit" de teinture comprenant au moins un premier compartiment contenant la composition tinctoriale définie ci-dessus et au moins un deuxième compartiment contenant une composition oxydante. Ce dispositif peut être équipé d'un moyen permettant de délivrer sur les cheveux le mélange souhaité, tel que les dispositifs décrits dans la demande de brevet FR-A-2 586 913.

Les exemples qui suivent servent à illustrer l'invention sans toutefois présenter un caractère limitatif.

## EXEMPLES

### EXEMPLE 1 : Compositions colorantes selon l'invention

Les compositions 1 et 2 suivantes selon l'invention ont été réalisées.

| Composition colorante | Composition 1 | Composition 2 |
|---|---|---|
| Cétyl hydroxyéthylcellulose (Polysurf 67 commercialisé par la société Aqualon) | 0,4 g | - |
| Cétyl hydroxyéthylcellulose (Natrosol Plus Grade 330 CS commercialisé par la société Aqualon) | - | 0,4 g |
| Hydroxypropylméthylcellulose | 0,19 g | 0,19 g |
| p-aminophénol | 0,1 g | - |
| p-phénylènediamine | - | 0,3 g |
| Diméthosulfate de 2,3-diaminodihydropyrazolo-pyrazolone | 1,9 g | - |
| Sulfate de 4,5-diamino-1-(β-hydroxyéthyl)pyrazole | - | 1,9 g |
| 4-amino-2-hydroxytoluène | 0,2 g | 1,4 g |
| 5-amino-6-chloro-o-crésol | 0,8 g | 0,2 g |
| Monoéthanolamide d'acide stéarique | 4,8 g | 4,8 g |

(suite)

| **Composition colorante** | **Composition 1** | **Composition 2** |
|---|---|---|
| Acide oléïque | 3 g | 3 g |
| Solution aqueuse à 20 % en poids en $NH_3$ | 7 g | 2,06 g |
| $TiO_2$ | 0,3 g | 0,3 g |
| Monoéthanolamine | 6,47 g | 6,28 g |
| Oleth-10 | 1,8 g | 1,8 g |
| Solution aqueuse à 40 % en poids Polyquaternium-6 (Merquat 100 commercialisé par la société Ondéo) | 1,6 g | 1,6 g |
| Acide éthylène diamine tétraacétique (EDTA) | 0,2 g | 0,2 g |
| Solution aqueuse à 60 % en poids de chlorure d'hexadiméthrine (Mexomère PO commercialisé par la société Chimex) | 1,2 g | 1,2 g |
| Oleth-30 | 1,5 g | 1,5 g |
| Stéareth-2 | 5,5 g | 5,5 g |
| Alcools en $C_{20}$-$C_{22}$ (Nafol 2022 EN commercialisé par la société Sasol) | 3 g | 3 g |
| Réducteur | q.s. | - |
| Eau déminéralisée q.s.p. | 100 g | 100 g |

**Protocole d'application**

Chaque composition 1 et 2 est diluée extemporanément, avec une fois et demie son poids d'une composition oxydante (pH voisin de 3) (eau oxygénée à 20 volumes) (6 % en poids d'$H_2O_2$). Le mélange se fait facilement et présente une bonne viscosité ; il est appliqué facilement sur des cheveux gris, à 90 % de cheveux blancs, à raison de 10 g pour 1 g de cheveux, pendant 30 minutes. Les cheveux sont ensuite rincés, lavés avec un shampooing standard et séchés.

La coloration capillaire est évaluée de manière visuelle. Les résultats obtenus sur les cheveux naturels gris, à 90 % de cheveux blancs, après traitement, sont les suivants :

| | **Nuance** |
|---|---|
| **Compostion 1** | Cuivré intense |
| **Composition 2** | Cuivré rouge intense |

Ces colorations possèdent de bonnes propriétés notamment en termes de sélectivité et de ténacité. Elles possèdent aussi une bonne intensité. Les compositions obtenues sont stables dans le temps.

**EXEMPLE 2 : exemple comparatif**

La composition 3 selon l'invention et la composition 4 comparative ont été réalisées.

| Composition colorante | **Composition 3 (invention)** | **Composition 4 (comparative)** |
|---|---|---|
| Hydroxyde d'ammonium | 10 g | 10 g |
| Acide érythorbique | 0,50 g | 0,50 g |
| Ethanolamine | 0,70 g | 0,70 g |
| EDTA | 0,20 g | 0,20 g |
| Sulfite de sodium | 0,50 g | 0,50 g |
| Dioxyde de titane | 0,30 g | 0,30 g |

(suite)

| Composition colorante | **Composition 3 (invention)** | **Composition 4 (comparative)** |
|---|---|---|
| 4-amino-2-hydroxytoluène | 0,25 g | 0,25 g |
| p-phénylènediamine | 0,22 g | 0,22 g |
| Alcool cétéarylique | 3 g | 3 g |
| Hydroxypropylméthylcellulose | 0,20 g | 0,20 g |
| Cétyl hydroxyéthylcellulose | 0,40 g | - |
| Polyuréthane cationique à chaine grasse obtenu à partir de la condensation de 1,3-bis(isocyanatométhylcyclohexane), de N,N-diméthyléthanolamine quaternisée avec le bromododécane, de N,N-diméthyléthanolamine et de polyoxyéthylène de poids moléculaire 10000 | - | 0,40 g |
| Acide oléique | 3 g | 3 g |
| Alcool stéarylique oxyéthyléné à 2 moles d'oxyde d'éthylène | 5,50 g | 5,50 g |
| Stéaramide monoéthanolamine, monoéthanolamine, acide stéarique (96:2:2) | 5 g | 5 g |
| Alcool oléocétylique oxyéthyléné à 30 moles d'oxyde d'éthylène | 1,50 g | 1,50 g |
| Eau | Qsp 100 g | Qsp 100 g |

**Protocole d'application**

Au moment de l'emploi, chacune des compositions 3 et 4 est mélangée avec une fois et demie son poids d'une composition oxydante (eau oxygénée à 20 volumes) (6 % en poids d'$H_2O_2$).

**Propriété rhéologique**

Les mesures sont réalisées sur les compositions 3 et 4, avant et après mélange avec ladite composition oxydante dans les conditions décrite ci-dessus.

Les mesures de viscosité sont effectuées à l'aide du rhéomètre Rhéomat RM 180 (Mettler) : vitesse de rotation 200 tr/min et température à 25°C avec un mobile différent selon les viscosités (mobile n°2, 3 ou 4, noté respectivement M1, M2 ou M3). Les viscosités sont mesurées 30 secondes après la mise en rotation du mobile.

**Résultats**

| | **Composition 3** | **Composition 4** |
|---|---|---|
| Aspect de la composition avant mélange | Crème épaisse | Crème fluide |
| Facilité de mélange avec la composition oxydante | Acceptable | Plus rapide |
| Répartition sur mèches | Bonne | Bonne mais composition un peu trop fluide |
| Viscosité de la composition avant mélange, en cps | 7100 au M4 | 700 au M3 |
| Viscosité de la composition oxydante en cps | 288 au M2 | |
| Viscosité de la composition après mélange avec la composition oxydante pendant 2min en cps | 1700 au M3 | 135 au M2 |

Unités de mesures en centipoises : cps

Avant et après mélange avec l'oxydant, la composition 3 selon l'invention est plus épaisse, et présente donc moins de risque de coulure lors de l'application

**Propriétés tinctoriales**

Chaque mélange est appliqué sur des mèches de cheveux à 90% de cheveux blancs naturelles (BN) et permanentées (BP) à raison de 15g de mélange par gramme de mèches de cheveux. Après 30 minutes de pause à température ambiante, les mèches sont rincées, lavées avec un shampooing standard, rincées à nouveau et séchées.
Les mesures colorimétriques sont réalisées à l'aide du spectrocolorimètre Konica Minolta CM-2600d dans le système CIE L*a*b*. Dans le système L* a* b*, L* représente l'intensité de la coloration obtenue, plus la valeur de L* est faible, plus la coloration obtenue est intense. La chromaticité est mesurée par les valeurs a* et b*, a* indiquant la valeur sur l'axe de couleur vert/rouge et b* indiquant la valeur sur l'axe de couleur bleu/jaune.
Pour chaque composition, on évalue la sélectivité de la coloration. La sélectivité de la coloration est la variation de la couleur entre des cheveux naturels et des cheveux permanentés. Les cheveux naturels sont représentatifs de la nature des cheveux à la racine alors que les cheveux permanentés sont représentatifs de la nature des cheveux à la pointe.
La sélectivité est mesurée par ΔE, qui est la variation de la couleur entre les cheveux naturels et les cheveux permanentés, est obtenu à partir de la formule :

$$\Delta E = \sqrt{(L^* - L_o{}^*)^2 + (a^* - a_o{}^*)^2 + (b^* - b_o{}^*)^2}$$

dans laquelle :

- L*, a* et b*, représentent les paramètres des cheveux permanentés colorés et
- $L_0$*, $a_0$* et $b_0$* représentent les paramètres des cheveux naturels colorés.

Plus la valeur de ΔE est faible, plus la sélectivité est faible et donc plus la coloration le long des cheveux est uniforme.

**Résultats**

| | Type cheveux | L* | a* | b* | ΔE |
|---|---|---|---|---|---|
| **Composition 3** | BN colorés | 22,19 | 14,57 | -0,30 | 3,96 |
| | BP colorés | 18,93 | 12,33 | -0,16 | |
| **Composition 4** | BN colorés | 26,25 | 16,83 | -0,31 | 5,99 |
| | BP colorés | 20,95 | 14,04 | -0,45 | |

Sur cheveux naturels et permanentés, la coloration obtenue avec la composition 3 selon l'invention est plus puissante (valeurs de L* plus faibles), que la coloration obtenue avec la composition 4.
La sélectivité (ΔE) est plus faible pour la composition 3 selon l'invention ce qui est représentatif d'une coloration plus uniforme le long de la fibre.

**Revendications**

1. Composition tinctoriale pour fibres kératiniques, comprenant, dans un milieu approprié pour la teinture :

   (A) un ou plusieurs dérivé(s) non ionique(s) de cellulose comprenant un ou plusieurs substituant(s) hydrophobe(s) comprenant de 8 à 30 atomes de carbone ;
   (B) un ou plusieurs polymère(s) non hydrophobe(s) non ionique(s) de type hydroxyalkylcellulose ; et
   (C) un ou plusieurs colorant(s) d'oxydation choisi(s) parmi les bases d'oxydation suivantes : les para-phénylènediamines, les bis-phénylalkylènediamines, les para-aminophénols, les bis-para-aminophénols, les ortho-aminophénols, et leurs sels d'addition.

2. Composition tinctoriale selon la revendication 1, **caractérisée en ce que** le dérivé non ionique de cellulose (A) est une hydroxyéthylcellulose substituée par un ou plusieurs substituant(s) hydrophobe(s) comprenant de 8 à 30 atomes

de carbone.

3. Composition tinctoriale selon l'une quelconque des revendications précédentes, **caractérisée en ce que** le substituant hydrophobe est un groupe alkyle en $C_{10}$-$C_{22}$.

4. Composition tinctoriale selon l'une quelconque des revendications précédentes, **caractérisée en ce que** le substituant hydrophobe est un groupement cétyle.

5. Composition tinctoriale selon l'une quelconque des revendications précédentes, **caractérisée en ce que** le degré de substitution hydrophobe va de 0,1 à 10 % en poids, de préférence de 0,1 à 1 % en poids et de manière plus préférée de 0,4 à 0,8 % en poids, du poids total du polymère.

6. Composition tinctoriale selon l'une quelconque des revendications précédentes, **caractérisée en ce que** la concentration en dérivé(s) non-ionique(s) de cellulose (A) va de 0,01 à 10 % en poids, de préférence de 0,05 à 3 % en poids et de manière plus préférée de 0,1 à 1 % en poids, par rapport au poids total de la composition.

7. Composition tinctoriale selon l'une quelconque des revendications précédentes, **caractérisée en ce que** le polymère non hydrophobe non ionique de type hydroxyalkylcellulose (B) comprend un ou plusieurs groupement(s) alkyle(s) ou hydroxyalkyle(s), identiques ou différents, linéaire(s) ou ramifié(s), saturé(s) ou insaturé(s), ayant de 1 à 4 atomes de carbone.

8. Composition tinctoriale selon l'une quelconque des revendications précédentes, **caractérisée en ce que** la concentration en polymère(s) non hydrophobe(s) non ionique(s) de type hydroxyalkylcellulose (B) va de préférence de 0,01 à 5 %, en particulier de 0,05 à 2 % en poids, et de manière plus préférée de 0,1 à 1% en poids, par rapport au poids total de la composition.

9. Composition tinctoriale selon l'une quelconque des revendications précédentes, **caractérisée en ce que** le colorant d'oxydation (C) comprend également un ou plusieurs coupleur(s) d'oxydation.

10. Composition tinctoriale selon la revendication précédente, **caractérisée en ce que** la concentration en base(s) d'oxydation ou la concentration en coupleur(s) d'oxydation va de 0,001 à 20 % en poids, de préférence de 0,005 à 10 % en poids, et encore plus préférentiellement de 0,01 à 5 % en poids, par rapport au poids total de la composition.

11. Composition tinctoriale selon l'une quelconque des revendications précédentes, **caractérisée en ce qu'**elle comprend un ou plusieurs colorant(s) direct(s) choisi(s) parmi les colorants nitrés benzéniques, les colorants directs azoïques, les colorants directs méthiniques, les colorants anthraquinoniques, les colorants xanthéniques, les colorants triarylméthaniques et leurs sels d'addition.

12. Composition tinctoriale selon l'une quelconque des revendications précédentes, **caractérisée en ce qu'**elle comprend au moins un agent oxydant.

13. Procédé de teinture d'oxydation des fibres kératiniques, **caractérisé en ce qu'**on applique sur les fibres une composition tinctoriale telle que définie dans l'une quelconque des revendications 1 à 11 en présence d'au moins un agent oxydant, pendant un temps suffisant pour développer la couleur désirée.

14. Dispositif à plusieurs compartiments, **caractérisé en ce qu'**il comprend au moins un premier compartiment contenant une composition tinctoriale telle que définie dans l'une quelconque des revendications 1 à 11 et au moins un deuxième compartiment contenant au moins un agent oxydant.

15. Utilisation de la composition définie dans l'une des revendications 1 à 12 pour la teinture des fibres kératiniques, en particulier, des fibres kératiniques humaines telles que les cheveux.

**Patentansprüche**

1. Färbezusammensetzung für Keratinfasern, die in einem zum Färben geeigneten Medium Folgendes umfasst:

(A) ein oder mehrere nichtionische(s) Zellulosederivat(e), umfassend einen oder mehrere hydrophobe(n) Substituenten, umfassend 8 bis 30 Kohlenstoffatome;
(B) ein oder mehrere nichthydrophobe(s) nichtionische(s) Polymer(e) des Hydroxyalkylzellulosetyps; sowie
(C) einen oder mehrere Oxidationsfarbstoff(e), ausgewählt aus den folgenden Oxidationsbasen : para-Phenylendiaminen, Bisphenylalkylendiaminen, para-Aminophenolen, Bis-para-Aminophenolen, ortho-Aminophenolen und ihren Additionssalzen.

**2.** Färbezusammensetzung nach Anspruch 1, **dadurch gekennzeichnet, dass** es sich bei dem nichtionischen Zellulosederivat (A) um eine durch einen oder mehrere hydrophobe(n) Substituenten, umfassend 8 bis 30 Kohlenstoffatome substituierte Hydroxyethylzellulose handelt.

**3.** Färbezusammensetzung nach einem der vorhergehenden Ansprüche, **dadurch gekennzeichnet, dass** es sich bei dem hydrophoben Substituenten um eine $C_{10}$-$C_{22}$-Alkylgruppe handelt.

**4.** Färbezusammensetzung nach einem der vorhergehenden Ansprüche, **dadurch gekennzeichnet, dass** es sich bei dem hydrophoben Substituenten um eine Cetylgruppe handelt.

**5.** Färbezusammensetzung nach einem der vorhergehenden Ansprüche, **dadurch gekennzeichnet, dass** der Grad der hydrophoben Substitution 0,1 bis 10 Gew.-%, vorzugsweise 0,1 bis 1 Gew.-% und am stärksten bevorzugt 0,4 bis 0,8 Gew.-% des Gesamtgewichts des Polymers beträgt.

**6.** Färbezusammensetzung nach einem der vorhergehenden Ansprüche, **dadurch gekennzeichnet, dass** die Konzentration an nichtionischem/n Zellulosederivat(en) (A) 0,01 bis 10 Gew.-%, vorzugsweise 0,05 bis 3 Gew.-% und am stärksten bevorzugt 0,1 bis 1 Gew.-% in Bezug auf das Gesamtgewicht der Zusammensetzung beträgt.

**7.** Färbezusammensetzung nach einem der vorhergehenden Ansprüche, **dadurch gekennzeichnet, dass** das nichthydrophobe nichtionische Polymer des Hydroxyalkylzellulosetyps (B) eine oder mehrere gleiche oder verschiedene, geradkettige oder verzweigte, gesättigte oder ungesättigte Alkyl- oder Hydroxyalkylgruppe(n) mit 1 bis 4 Kohlenstoffatomen umfasst.

**8.** Färbezusammensetzung nach einem der vorhergehenden Ansprüche, **dadurch gekennzeichnet, dass** die Konzentration an nichthydrophobem/n nichtionischem/n Polymer(en) des Hydroxyalkylzellulosetyps (B) vorzugsweise 0,01 bis 5 Gew.-%, insbesondere 0,05 bis 2 Gew.-%, am stärksten bevorzugt 0,1 bis 1 Gew.-%, in Bezug auf das Gesamtgewicht der Zusammensetzung beträgt.

**9.** Färbezusammensetzung nach einem der vorhergehenden Ansprüche, **dadurch gekennzeichnet, dass** der Oxidationsfarbstoff (C) auch einen oder mehrere Oxidationskuppler umfasst.

**10.** Färbezusammensetzung nach dem vorhergehenden Anspruch, **dadurch gekennzeichnet, dass** die Konzentration an Oxidationsbase(n) oder die Konzentration an Oxidationskuppler(n) 0,001 bis 20 Gew.-%, vorzugsweise 0,005 bis 10 Gew.-%, noch stärker bevorzugt 0,01 bis 5 Gew.-%, in Bezug auf das Gesamtgewicht der Zusammensetzung beträgt.

**11.** Färbezusammensetzung nach einem der vorhergehenden Ansprüche, **dadurch gekennzeichnet, dass** sie einen oder mehrere direkte(n) Farbstoffe(n), ausgewählt aus der Reihe der Nitrobenzolfarbstoffe, der direkten Azofarbstoffe, der direkten Methinfarbstoffe, der Anthrachinonfarbstoffe, der Xanthenfarbstoffe, der Triarylmethanfarbstoffe und ihren Additionssalzen umfasst.

**12.** Färbezusammensetzung nach einem der vorhergehenden Ansprüche, **dadurch gekennzeichnet, dass** sie mindestens ein Oxidationsmittel umfasst.

**13.** Verfahren für die Oxidationsfärbung von Keratinfasern, **dadurch gekennzeichnet, dass** man eine Färbezusammensetzung wie in einem der Ansprüche 1 bis 11 definiert in Gegenwart von mindestens einem Oxidationsmittel über einen Zeitraum, der ausreicht, um die gewünschte Farbe zu entwickeln, auf die Fasern aufbring.

**14.** Einheit mit mehreren Kompartimenten, **dadurch gekennzeichnet, dass** sie mindestens ein erstes Kompartiment umfasst, das eine Färbezusammensetzung wie in einem der Ansprüche 1 bis 11 definiert enthält, und mindestens ein zweites Kompartiment, das mindestens ein Oxidationsmittel enthält, umfasst.

**15.** Verwendung der in einem der Ansprüche 1 bis 12 definierten Zusammensetzung zum Färben von Keratinfasern, insbesondere menschlichen Keratinfasern wie dem Haar.

## Claims

**1.** Dye composition for keratin fibres, comprising, in a medium suitable for dyeing:

(A) one or more nonionic derivative(s) of cellulose comprising one or more hydrophobic substituent(s) containing from 8 to 30 carbon atoms;
(B) one or more nonionic nonhydrophobic polymer(s) of hydroxyalkylcellulose type; and
(C) one or more oxidation dye(s) chosen from the following oxidation bases: para-phenylenediamines, bisphenylalkylenediamines, para-aminophenols, bis-para-aminophenols, ortho-aminophenols, and addition salts thereof.

**2.** Dye composition according to Claim 1, **characterized in that** the nonionic derivative of cellulose (A) is a hydroxyethylcellulose substituted with one or more hydrophobic substituent(s) containing from 8 to 30 carbon atoms.

**3.** Dye composition according to any one of the preceding claims, **characterized in that** the hydrophobic substituent is a $C_{10}$-$C_{22}$ alkyl group.

**4.** Dye composition according to any one of the preceding claims, **characterized in that** the hydrophobic substituent is a cetyl group.

**5.** Dye composition according to any one of the preceding claims, **characterized in that** the degree of hydrophobic substitution ranges from 0.1 to 10% by weight, preferably from 0.1 to 1% by weight and more preferably from 0.4 to 0.8% by weight, relative to the total weight of the polymer.

**6.** Dye composition according to any one of the preceding claims, **characterized in that** the concentration of nonionic derivative(s) of cellulose (A) ranges from 0.01 to 10% by weight, preferably from 0.05 to 3% by weight and more preferably from 0.1 to 1% by weight, relative to the total weight of the composition.

**7.** Dye composition according to any one of the preceding claims, **characterized in that** the nonionic nonhydrophobic polymer of hydroxyalkylcellulose type (B) comprises one or more identical or different, linear or branched, saturated or unsaturated, alkyl or hydroxyalkyl group(s) containing from 1 to 4 carbon atoms.

**8.** Dye composition according to any one of the preceding claims, **characterized in that** the concentration of nonionic nonhydrophobic polymer(s) of hydroxyalkylcellulose type (B) preferably ranges from 0.01 to 5%, in particular from 0.05 to 2% by weight, more preferably from 0.1 to 1% by weight, relative to the total weight of the composition.

**9.** Dye composition according to any one of the preceding claims, **characterized in that** the oxidation dye (C) also comprises one or more oxidation coupler(s).

**10.** Dye composition according to the preceding claim, **characterized in that** the concentration of oxidation base(s) or the concentration of oxidation coupler(s) ranges from 0.001 to 20% by weight, preferably from 0.005 to 10% by weight, and even more preferentially from 0.01 to 5% by weight, relative to the total weight of the composition.

**11.** Dye composition according to any one of the preceding claims, **characterized in that** it comprises one or more direct dye(s) chosen from nitrobenzene dyes, azo direct dyes, methine direct dyes, anthraquinone dyes, xanthene dyes and triarylmethane dyes, and addition salts thereof.

**12.** Dye composition according to any one of the preceding claims, **characterized in that** it comprises at least one oxidizing agent.

**13.** Process for the oxidation dyeing of keratin fibres, **characterized in that** a dye composition as defined in any one of Claims 1 to 11 is applied to the fibres in the presence of at least one oxidizing agent, for a period of time sufficient to develop the desired colour.

**14.** Multicompartment device, **characterized in that** it comprises at least a first compartment containing a dye composition as defined in any one of Claims 1 to 11 and at least a second compartment containing at least one oxidizing agent.

**15.** Use of the composition defined in one of Claims 1 to 12, for dyeing keratin fibres, in particular human keratin fibres such as the hair.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9803150 A
- EP 1426040 A
- FR 2586913 A